# FASCICULE DE BREVET EUROPEEN

(11) **EP 3 024 924 B1**
(45) Date de publication et mention de la délivrance du brevet: **24.07.2019**
(21) Numéro de dépôt: 14755879.5
(22) Date de dépôt: 25.07.2014
(51) Int. Cl.: C12N 1/12

(54) **PROCÉDÉ DE FERMENTATION DE CHLORELLES EN MODE DISCONTINU ALIMENTE PAR APPORTS SÉQUENTIELS ET AUTOMATISES EN GLUCOSE**
VERFAHREN FÜR FED-BATCH-FERMENTATION VON CHLORELLAE DURCH SEQUENZIELLE AUTOMATISIERTE GLUCOSEZUFUHR
METHOD FOR FED-BATCH FERMENTATION OF CHLORELLAE FED BY SEQUENTIAL, AUTOMATED PROVISIONS OF GLUCOSE

(30) Priorité: 26.07.2013 FR 1357387
(43) Date de publication de la demande: 01.06.2016
(73) Titulaire: Corbion Biotech, Inc., South San Francisco, CA 94080 (US)
(72) Inventeur: SEGUEILHA, Laurent, F-59520 Marquette Lez Lille (FR); LE RUYET, Marie, F-59000 Lille (FR); DELAROCHE, Sylvain, F-62219 Longuenesse (FR)
(74) Mandataire: Cabinet Becker et Associés
(86) Numéro de dépôt international: PCT/FR2014/051943
(87) Numéro de publication internationale: WO 2015/011428

(56) Documents cités:
- EP-A1- 1 359 224
- WO-A2-2014/074769
- US-A- 5 912 113
- PEREZ-GARCIA O ET AL: "Heterotrophic cultures of microalgae: Metabolism and potential products", WATER RESEARCH, ELSEVIER, AMSTERDAM, NL, vol. 45, no. 1, 1 janvier 2011 (2011-01-01), pages 11-36, XP027536678, ISSN: 0043-1354 [extrait le 2010-08-27]
- SANSAWA H ET AL: "Production of intracellular phytochemicals in Chlorella under heterotrophic conditions", JOURNAL OF BIOSCIENCE AND BIOENGINEERING, ELSEVIER, AMSTERDAM, NL, vol. 98, no. 6, 1 janvier 2004 (2004-01-01), pages 437-444, XP004727088, ISSN: 1389-1723

## Description

La présente invention est relative à un nouveau procédé de production de microalgues du genre *Chlorella* par fermentation en mode discontinu alimenté (dite « fed Batch »)

### Présentation de l'état de l'art

Ne nécessitant historiquement « que de l'eau et de la lumière du soleil » pour croître, les algues sont considérées depuis longtemps comme une source de nourriture.

Il existe plusieurs espèces d'algues pouvant être utilisées en alimentation, la plupart étant des "macroalgues" comme le varech, la laitue de mer (*Ulva lactuca*) et des algues rouges de type *Porphyra* (cultivée au Japon) ou « dulse » (*Palmaria palmata*).

Cependant, outre ces macroalgues, il y a aussi d'autres sources d'algues représentées par les « microalgues », c'est à dire des algues microscopiques unicellulaires photosynthétiques ou non, d'origine marine ou non, cultivées pour leurs applications dans les biocarburants ou l'alimentation.

Par exemple, la spiruline (*Arthrospira platensis*) est cultivée dans des lagunes ouvertes (par phototrophie) pour une utilisation comme complément alimentaire ou incorporée en petites quantités dans la confiserie ou les boissons (généralement moins de 0,5% poids / poids).

D'autres microalgues riches en lipides, y compris certaines espèces appartenant au genre *Chlorella,* sont aussi très populaires dans les pays asiatiques comme compléments alimentaires.

Plusieurs espèces de microalgues sont capables de passer d'une croissance photo-autotrophe (grâce à de la lumière qui fournit l'énergie pour convertir le CO₂ en chaînes carbonées) à une croissance hétérotrophe (sans lumière) utilisant le glucose ou d'autres substrats carbonés utilisables pour le métabolisme du carbone et de l'énergie.

Industriellement, trois procédés de production des microalgues sont actuellement utilisés :
- En réacteurs hétérotrophes (entièrement clos) ;
- En bassins à ciel ouvert ;
- En tubes de verre

De ces modes de culture sont produites des Chlorelles aux propriétés et compositions variables. Selon qu'elles sont produites à la lumière ou non, à l'air libre ou pas, leur composition sera différente.

La production et l'utilisation de la farine de microalgues de type *Chlorella* sont par exemple décrits dans les documents WO 2010/120923 et WO 2010/045368.

La fraction huile de la farine de microalgues, qui peut être composée essentiellement d'huiles monoinsaturées, peut offrir des avantages nutritionnels et santé par rapport aux huiles saturées, hydrogénées et polyinsaturés souvent trouvées dans les produits alimentaires conventionnels.

Lorsque l'on souhaite fabriquer industriellement des poudres de farine de microalgues à partir de leur biomasse, d'importantes difficultés demeurent, non seulement du point de vue technologique, mais également du point de vue du profil sensoriel des compositions produites.

En effet, si des poudres d'algues par exemple fabriquées avec des algues cultivées photosynthétiquement dans des étangs extérieurs ou par photobioréacteurs sont disponibles dans le commerce, elles ont une couleur vert foncée (liée à la chlorophylle) et un goût fort, désagréable.

Or, il est ainsi généralement admis que la formation et le développement des chloroplastes sont supprimés en conditions de culture hétérotrophiques et à l'obscurité.

Dans ces conditions hétérotrophiques, les microalgues n'utilisent donc pas la réaction de photosynthèse mais se développent en consommant les sucres du milieu de culture.

Les avantages de ce système de production sont :
- une productivité volumique fortement augmentée, multipliée par 100 par rapport à un système ouvert, et par 10 par rapport aux photobioréacteurs,
- des concentrations en matière sèche très importantes (des centaines de grammes par litre),
- des coûts de production faibles,
- des produits obtenus de très grande qualité,
- un milieu confiné donc pas de contamination,
- pas de contrainte de localisation,
- très industrialisable,
- une technologie totalement maîtrisée à l'échelle industrielle sur les levures et bactéries depuis plusieurs décennies, par différentes filières comme la chimie et l'agro-alimentaire,
- absence de chlorophylle et donc goût plus neutre.

Deux modes de culture hétérotrophique sont classiquement décrits dans la littérature (par exemple par H. IWAMOTO dans Richmond, A. (ed.), 2004, Handbook of microalgal culture. Blackwell, Oxford, 255-263*)..*

H. IWAMOTO écrit que lorsque l'étape de culture hétérotrophique est contrôlée en mode « batch » (apport de tout le glucose en une seule fois en début de fermentation), la phase initiale de croissance exponentielle est suivie d'une étape de maturation, de manière à obtenir des cellules riches en composés d'intérêt.

La biomasse augmente durant la phase initiale, avec consommation du glucose, puis s'arrête à glucose zéro.

La seconde phase dite de maturation est mise à profit ensuite pour favoriser la production d'autres molécules d'intérêt (pigments, lipides...).

La culture en mode « fed-batch » (alimentation progressive en glucose) est généralement mise en oeuvre en conditions dites limitantes en glucose.

Le principe au coeur de la méthode, tel que décrit par H. IWAMOTO implique l'apport du glucose en réponse à sa consommation par les chlorelles en croissance.

La concentration du glucose dans le milieu de culture est analysée de manière continue et automatique, et est maintenue à 1,5 %.

On arrête l'alimentation en glucose lorsque la densité cellulaire souhaitée est atteinte. La culture est alors maintenue dans cet état environ 10 heures afin de favoriser la maturation cellulaire.

L'application de ce mode de culture fed batch sous conditions limitantes en glucose est illustrée par H. IWAMOTO avec une souche de *Chlorella regularis.*

L'utilisation d'une culture en fed-batch de *Chlorella regularis* dans laquelle l'apport en glucose est contrôlé par la mesure de la concentration en glucose dans le milieu, est également illustrée dans l'article de Sansawa et al. (Journal of Bioscience and Bioengineering, vol.98, No. 6, 437-444, 2004). Un procédé de culture fed-batch de microorganismes marins, en particulier de dinoflagellés, produisant de l'acide docosahexaénoïque, est par ailleurs décrit dans la demande de brevet EP 1 359 224.

Chez H. IWAMOTO, la culture est menée pendant une durée totale de 40 h, les 30 premières heures étant consacrées à la croissance des microalgues et leur alimentation en glucose.

Les 10 heures suivantes sont dédiées à la maturation, sans apport de glucose.

La biomasse est alors collectée et concentrée par centrifugation, lavage, inactivation thermique (permettant d'inhiber la chlorophylase) à 130°C pendant 3 secondes, et séchées par atomisation, afin d'obtenir une poudre très fine.

Cependant, cette conduite est surtout mise en oeuvre pour réactiver la production de chlorophylle et de caroténoïdes pendant l'étape de maturation.

Elle n'est pas adaptée à la production de biomasse de *Chlorella* riches en lipides sans défaut altérant les qualités organoleptiques (terme anglo-saxon de « off-notes »), et en particulier sans production de chlorophylle.

Par ailleurs, les appareils de dosage automatiques du glucose résiduel ne sont pas suffisamment fiables et ne donnent pas de réponse assez rapide (> 1 minute) pour permettre une régulation fine de la fermentation.

Il demeure donc un besoin non satisfait de disposer d'une méthode de production efficace par fermentation fed Batch qui soit libérée des contraintes de gestion du glucose résiduel.

### Résumé de l'invention

La société Demanderesse a trouvé que l'on pouvait répondre à ce besoin en proposant un procédé de production de microalgues du genre *Chlorella,* par fermentation fed batch dans laquelle les ajouts de source carbonée sont séquentiels et automatisés dans des conditions tout à fait particulières, c'est-à-dire en asservissant l'apport en source carbonée par ajouts séquentiels, à la valeur de la pression en oxygène dissous (pO₂) du milieu de fermentation.

La présente invention concerne donc un procédé de production de microalgues du genre *Chlorella* par fermentation en mode discontinu alimenté (ou « fed Batch »), caractérisé en ce que l'apport en source carbonée est réalisé de manière séquentielle et automatique en réponse à une baisse de la consommation en oxygène par la microalgue.

L'objet de la présente invention est défini par les revendications.

La microalgue peut être choisie dans le groupe constitué de *Chlorella protothecoides, Chlorella sorokiniana* et *Chlorella vulgaris.* De préférence, la microalgue est *Chlorella protothecoides.*

La source carbonée est le glucose.

La baisse de la consommation en oxygène de la microalgue est détectée en mesurant la pression en oxygène dissous dans le milieu. Une augmentation de cette pression traduisant une baisse de la consommation, l'apport en source carbonée est déclenché lorsque la pression en oxygène dissous dans le milieu de fermentation (pO₂) dépasse une valeur seuil.

Cette valeur seuil est de 10 à 80 % supérieure à la pO₂ dans le milieu de fermentation lorsque la concentration en source carbonée dans le milieu de fermentation n'est pas limitante.

Lors du déclenchement, le glucose est apporté durant une période de temps de moins de 10 minutes pour atteindre une concentration de 1 à 20 g/L de glucose dans le milieu de fermentation.

De préférence, la valeur de pO₂ établie lorsque la concentration en source carbonée dans le milieu de fermentation n'est pas limitante, est de 20 à 40%, et de manière plus particulièrement préférée d'environ 30%.

De préférence le délai entre le moment où la source de carbone a été entièrement consommée et un apport en source carbonée est inférieur à 5 minutes, de manière plus particulièrement préférée, inférieur à 1 minute.

La source de carbone résiduelle est de préférence maintenue en permanence, ou de façon quasi permanente, à une valeur supérieure à 0 et inférieure à 20g/L, de préférence inférieure à 10 g/L.

De préférence, l'apport en source carbonée est réalisée au moyen d'une pompe dont le débit maximal permet d'ajouter 10 à 20 g/L de source carbonée au milieu de fermentation en moins de 10 minutes.

### Description détaillée de l'invention

Contrairement au préjugé technique qui veut que, dans le cas d'une fermentation en fed-batch, l'on doive maintenir la concentration en glucose résiduel entre 5 et 15 g/l par un ajout continu en glucose régulé par la mesure automatique de la concentration en glucose résiduel dans le milieu, la société Demanderesse tire profit de l'observation selon laquelle lorsque le glucose dans le milieu de fermentation a été entièrement consommé, la pO₂ augmente rapidement.

La société Demanderesse automatise ainsi les apports en source carbonée en programmant la pompe d'alimentation en glucose afin que celle-ci déclenche le pulse de glucose à chaque remontée de pO₂ et non pas lorsque la concentration en glucose résiduel est en deçà des 5 g/l.

En d'autres termes, la pompe d'alimentation en glucose s'enclenche dès que la valeur mesurée de la pO₂ est supérieure à une valeur seuil établie par rapport à la pO₂ mesurée lorsque la concentration en glucose résiduel n'est pas limitante.

Cela se traduit par le fait que la détection de la remontée de la pO₂ au-delà d'un seuil déterminé déclenche la mise en route, de préférence à sa vitesse maximale, de la pompe d'alimentation en glucose pendant un temps déterminé (de préférence moins de 10 minutes) pour apporter une quantité de glucose correspondant à une concentration comprise entre 1 et 30 g/L, de préférence environ 10 g/L, ramenée au volume initial du fermenteur.

Grâce à ce procédé, c'est la microalgue qui « gère » directement et automatiquement ses besoins en glucose.

Par rapport au procédé de fermentation en fed-batch avec une alimentation continue en source carbonée, le procédé selon l'invention présente plusieurs avantages :
- il ne nécessite aucune intervention humaine étant donné qu'il permet une régulation fine et automatique de la fermentation, comme cela est démontré dans la partie expérimentale ;
- les apports en source carbonée sont réalisés selon les besoins des microorganismes. Ainsi, le milieu de fermentation ne peut en aucun cas accumuler une quantité de glucose supérieure à celle définie par les pulses ou à l'opposé, se retrouver longtemps à glucose résiduel nul. Ces deux paramètres sont primordiaux pour assurer la qualité du produit fini notamment l'absence d'« off-notes », c'est à-dire de caractéristiques organoleptiques altérées ;
- le glucose résiduel est bien mieux maîtrisé, le protocole de fermentation est donc plus robuste et plus reproductible ;
- la productivité est maximale, sans sous-alimentation en glucose et sans accumulation de glucose à des concentrations inhibitrices pour le métabolisme de la microalgue.

Tel qu'utilisé ici, le terme *«productivité»* correspond à la quantité de biomasse fabriquée par litre et par heure de fermentation en mode fed batch.

*Le rendement de conversion Yx*/*s* représente classiquement le rapport entre la biomasse formée et le glucose consommé. Cependant, au sens de l'invention, afin de comparer les différents protocoles testés, notamment dans la partie expérimentale, et évaluer l'impact des modifications sur le rendement, la société Demanderesse a choisi de rationaliser ce paramètre en déterminant la valeur de rendement pour la production de 45 % en acides gras produits (en p/p sec de biomasse).

La présente invention concerne donc un procédé de production de microalgues du genre *Chlorella* par fermentation en mode discontinu alimenté (ou « fed Batch »), caractérisé en ce que l'apport en source carbonée est réalisé de manière séquentielle et automatique en réponse à une baisse de la consommation en oxygène par la microalgue.

La source carbonée est le glucose.

Les termes « fermentation en mode discontinu alimenté » ou « fermentation fed-batch » peuvent être utilisés de manière interchangeable. Dans ce mode de fermentation, après un début de fermentation en batch, c'est-à-dire sans apport, une source carbonée est ajoutée tout au long du processus de fermentation jusqu'à obtention d'une quantité de biomasse définie.

Contrairement aux procédés fed-batch de l'art antérieur dans lesquels l'apport en source carbonée se fait par une alimentation continue, dans le procédé selon l'invention l'apport en source carbonée se fait par des ajouts séquentiels ou « puises ».

Ces ajouts séquentiels ou « puises » consistent pour chacun en l'ajout d'une quantité importante d'une solution concentrée de la source carbonée, de préférence un sirop de glucose, en un temps relativement court, inférieur à 10 min, et de manière particulièrement préférée d'environ 6 min, pour atteindre la concentration souhaitée dans le moût de fermentation. La concentration souhaitée dans le moût de fermentation est comprise entre 1 et 20 g/L et de préférence entre 10 et 20 g/L. Selon un mode de réalisation particulier, la concentration souhaitée de source carbonée dans le moût de fermentation est d'environ 10 g/L. Tel qu'utilisé ici, le terme « environ » se réfère à une valeur +/- 20%, 10%, 5% ou 2%.

Comme il sera exemplifié ci-après, dès que le glucose dans le milieu est entièrement consommé et que par conséquent la pO₂ augmente, on alimente avec une solution de glucose concentrée, par exemple à 700 g/L, pendant un laps de temps de moins de 10 minutes, pour atteindre une concentration de 1 à 20 g/L de glucose dans le milieu de fermentation. L'apport de glucose est ensuite stoppé pendant la durée de consommation du glucose résiduel. Lorsque celui-ci est consommé, la pO₂ remonte ce qui déclenche un nouvel apport en glucose, et ainsi de suite.

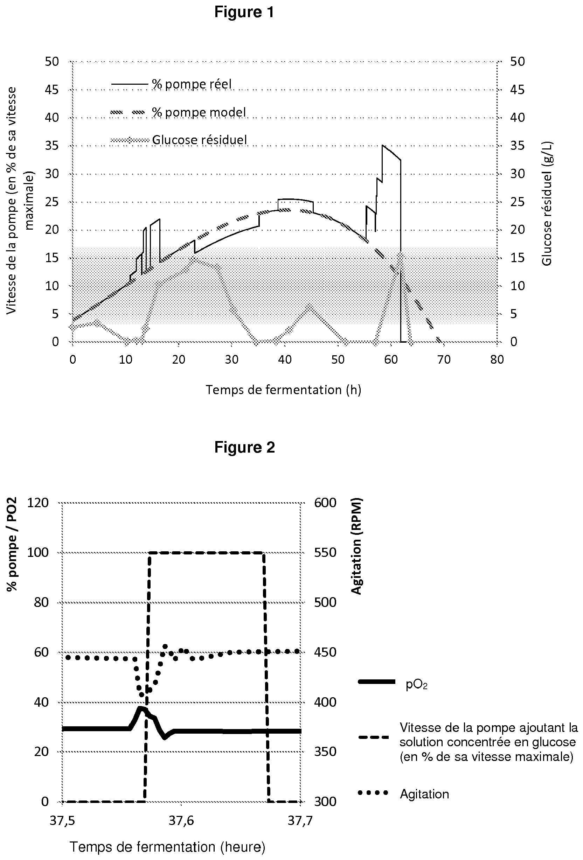

Comme mentionné précédemment, ces ajouts ne nécessitent pas d'intervention humaine particulière contrairement à un ajout continu durant lequel il faut s'assurer que le débit d'alimentation en glucose correspond bien au besoin métabolique de la souche (cf. Figure 1). En effet, une fois le glucose consommé, la consommation en oxygène du milieu de fermentation par la microalgue va se réduire brusquement. Ainsi suivant les réglages PID (« proportionnel intégral dérivé ») de l'agitation ou du débit d'air ou de la contrepression de dôme ou de l'apport d'O₂, une remontée significative de quelques % de la pO₂ va s'opérer. Dans le procédé selon l'invention, c'est cette remontée qui déclenche automatiquement un apport en glucose.

L'apport en source carbonée est réalisée au moyen d'une pompe dont le débit maximal permet de préférence d'ajouter 10 à 20 g/L de source carbonée au milieu de fermentation en moins de 10 minutes.

L'apport en source carbonée est réalisé dès que la pO₂ dépasse une valeur seuil qui est de 10 à 80%, ou de préférence de 15 à 70%, supérieure à la valeur de pO₂ établie lorsque la concentration en source carbonée dans le milieu de fermentation n'est pas limitante. Selon un mode particulier de réalisation, l'apport en source carbonée est réalisé dès que la pO₂ dépasse une valeur seuil qui est de 10 à 20 %, et de préférence d'environ 15%, supérieure à la valeur de pO₂ établie lorsque la concentration en source carbonée dans le milieu de fermentation n'est pas limitante. Selon un autre mode particulier de réalisation, l'apport en source carbonée est réalisé dès que la pO₂ dépasse une valeur seuil qui est de 50 à 80 %, plus préférentiellement encore de 60 à 70 %, et de manière plus particulièrement préférée d'environ 65%, supérieure à la valeur de pO₂ établie lorsque la concentration en source carbonée dans le milieu de fermentation n'est pas limitante.

A titre d'exemple, une concentration en glucose non limitante peut être une concentration comprise entre 5 et 15 g/L. La valeur de la pO₂ lorsque la concentration en source carbonée dans le milieu de fermentation n'est pas limitante, dépend de plusieurs paramètres dont les réglages PID (« proportionnel intégral dérivé ») de l'agitation ou du débit d'air ou de la contrepression de dôme ou de l'apport d'O₂. Ces paramètres sont de préférence constants tout au long de la phase de fermentation destinée à augmenter la biomasse. Ainsi, les variations de pO₂ traduisent fidèlement les variations de consommation en oxygène de la microalgue.

La valeur de la pO₂ lorsque la concentration en source carbonée dans le milieu de fermentation n'est pas limitante est habituellement de 20 à 40%, de préférence d'environ 30 %.

Selon un mode de réalisation particulier, la valeur de pO₂ lorsque la concentration en source carbonée dans le milieu de fermentation n'est pas limitante, est d'environ 30%, de préférence 30%, et la valeur seuil déclenchant un apport en source de carbone est comprise entre environ 35% et environ 55%, de préférence entre 35% et 55%. Selon un autre mode de réalisation particulier, la valeur de pO₂ lorsque la concentration en source carbonée dans le milieu de fermentation n'est pas limitante, est d'environ 30%, de préférence 30%, et la valeur seuil déclenchant un apport en source de carbone est d'environ 35%, de préférence 35%.

De préférence, le délai entre le moment où la source de carbone a été entièrement consommée et l'apport en source carbonée dans le milieu de fermentation est inférieur à 5 minutes, de manière tout particulièrement préférée inférieur à 1 minute. Comme indiqué ci-dessus, la consommation complète de la source carbonée entraine une baisse brutale de la consommation en oxygène qui est détectée par la mesure de la valeur de la pO₂ dans le milieu de fermentation au moyen d'une sonde spécifique, une augmentation de la pO₂ traduisant une baisse de la consommation en oxygène et donc une carence en source de carbone dans le milieu.

Ce temps de réaction permet donc de maintenir la source de carbone résiduelle de façon quasi constante à une valeur supérieure à 0 et inférieure à 20 g/L, de préférence supérieure à 0 et inférieure à 10 g/L, sans risque de limitation ou d'inhibition du métabolisme par le glucose.

La microalgue peut être toute *Chlorella* adaptée à une fermentation en fed-batch. Selon un mode de réalisation, la microalgue est choisie dans le groupe constitué de *Chlorella protothecoides, Chlorella sorokiniana* et *Chlorella vulgaris.* De préférence, la microalgue est *Chlorella protothecoides.* Selon un mode de réalisation particulier, la microalgue est *Chlorella protothecoides* UTEX 250 (The Culture Collection of Algae at the University of Texas at Austin - USA).

L'invention sera mieux comprise à l'aide des exemples qui suivent, lesquels se veulent illustratifs et non limitatifs.

### EXEMPLES

### Exemple 1. Production de Chlorella protothecoides riche en lipide à l'aide de deux méthodes d'apport du glucose

La souche utilisée est *Chlorella protothecoides UTEX 250 (The Culture Collection of Algae at the University of Texas at Austin* - USA)

### Préculture :

- 500 mL de milieu dans un Erlenmeyer de 2L ;
- Composition du milieu (en g/L) :

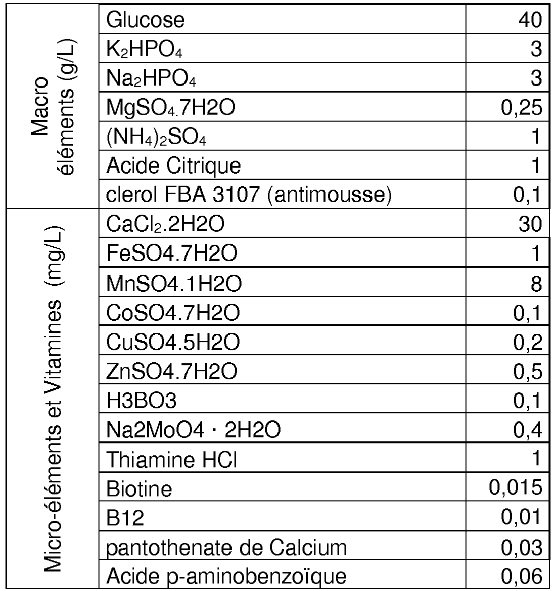

L'incubation se déroule dans les conditions suivantes : durée : 72 h ; température : 28°C ; agitation : 110 rpm (Incubateur Infors Multitron).

La préculture est ensuite transférée dans un fermenteur de 30L de type Sartorius.

### Culture pour production de biomasse :

Le milieu de base est le suivant :

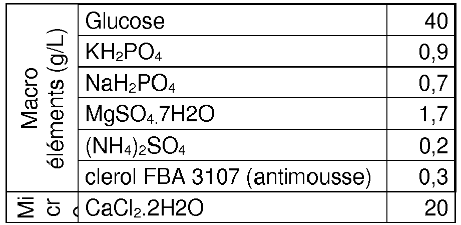

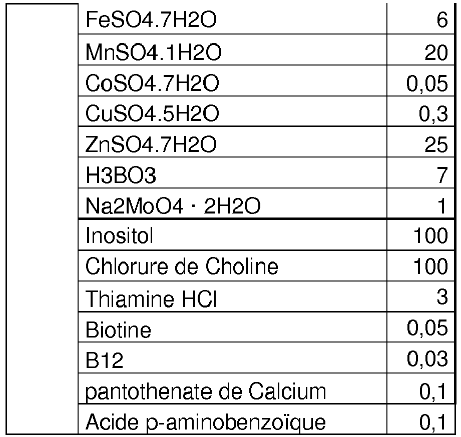

Le volume initial (Vi) du fermenteur est ajusté à 7 L après ensemencement. Il est porté à 15 - 20 L en final.

Les paramètres de conduite de la fermentation sont les suivants :

| | |
|---|---|
| Température | 28 °C |
| pH | 6.8 (essais 1 et 2) ou 5.2 (essais 3 et 4) par NH3 28% w/w puis KOH 5N |
| pO₂ | 30 % (maintenue par agitation) |
| Agitation | 300 RPM mini |
| Débit d'air | 15 L/min |

### Mode d'apport du glucose

De manière classique, lorsque la concentration résiduelle en glucose tombe en dessous de 10 g/L, un apport de glucose est réalisé de façon à maintenir la teneur en glucose entre 0 et 20 g/L dans le fermenteur.

Cet apport est réalisé à partir d'une solution de glucose concentrée à 700 g/L qui est transférée dans le fermenteur à l'aide d'une pompe péristaltique.

L'apport en glucose est géré selon deux méthodes différentes :

### 1) Protocole 1 : apport de glucose en continu avec vitesse ajustée manuellement (essais 1 et 3)

Dans ce type d'alimentation, la pompe fonctionne en permanence.

L'évolution des besoins de la souche en glucose au cours de la fermentation ayant été modélisée, un profil d'évolution modèle du débit de la pompe est programmé (cf. Figure 1, courbe « % pompe model »).

Mais, les débits (de l'ordre de 20 g/L/h au maximum) et les quantités cumulées de glucose (environ 1000 g/L) étant très forts, un faible écart (inférieur à la précision du modèle) entre le débit appliqué et la vitesse réelle de consommation de la souche entraine rapidement une forte variation du glucose résiduel.

Comme le montre la figure 1, des ajustements manuels sont donc fréquemment nécessaires (cf. Figure 1, courbe « % pompe réel ») en cours de culture pour maintenir le glucose résiduel (cf. Figure 1, courbe « glucose résiduel ») dans la gamme voulue sans pouvoir obtenir une stabilité parfaite.

Cette méthode réclame donc un contrôle permanent par un opérateur et le métabolisme de la souche est parfois limité par la disponibilité du glucose.

### 2) Protocole 2 : apport de glucose par ajouts séquentiels et automatiques en fonction de la pO₂ (essais 2 et 4)

Par le procédé conforme à l'invention, les ajouts de glucose sont séquentiels et automatisés grâce à un algorithme contrôlant le fonctionnement de la pompe à partir de la mesure de la teneur en oxygène dissous (pO₂) dans le milieu de fermentation à l'aide d'une sonde dédiée.

Le principe du contrôle est présenté dans la figure 2.

Lorsque la concentration en glucose tombe à 0 g/L dans le fermenteur, la consommation en oxygène de la souche chute fortement de sorte que la pO₂ remonte rapidement malgré la baisse de l'agitation qui est déclenchée par l'algorithme de régulation de la pO₂.

La détection de la remontée de la pO₂ au-delà d'un seuil déterminé (35 %) déclenche la mise en route de la pompe d'alimentation en glucose à sa vitesse maximale pendant un temps déterminé (6 minutes) pour apporter une quantité de glucose correspondant à une concentration choisie (10 g/L ramenée au volume initial du fermenteur).

Dans cet exemple, le délai entre la consommation complète du glucose et le nouvel ajout est inférieur à une minute.

Le glucose résiduel est donc en permanence maintenu à une valeur supérieure à 0 et inférieure à 10 g/L, sans risque de limitation ou d'inhibition du métabolisme par le glucose.

Contrairement au protocole manuel, la vitesse de la fermentation n'est à aucun moment ralentie par le glucose.

De plus, cette méthode est entièrement automatisée et fonctionne sans qu'une surveillance humaine ne soit nécessaire.

### Phase d'enrichissement en lipides

Dans tous les cas, lorsque 1000 g de glucose ont été consommés et que la biomasse a atteint une concentration de 70 g/L, l'ammoniaque est remplacé par de la potasse pour la régulation du pH. Cela permet à la biomasse d'accumuler des lipides

### Résultats:

| Essai | Alimentation Glucose | Concentration en Glucose résiduelle (g/L) | pH | Durée (h) | Biomasse (g/L) | % Lipides |
|---|---|---|---|---|---|---|
| 1 | Ajout continu avec ajustements manuels (Protocole 1) | 0 - 30 | 6,8 | 96 | 177 | 45,4 |
| 2 | Ajouts séquentiels et automatiques (Protocole 2) | 0 - 10 | 6,8 | 86 | 175 | 45,3 |
| 3 | Ajout continu avec ajustements manuels (Protocole 1) | 0 - 30 | 5,2 | 94,5 | 182 | 44,6 |
| 4 | Ajouts séquentiels et automatiques (Protocole 2) | 0 - 10 | 5,2 | 88,8 | 176 | 44,5 |

Le protocole 2 selon l'invention, c'est-à-dire l'apport de glucose par ajouts séquentiels et automatiques, permet d'augmenter la productivité. En effet, il faut moins de temps de fermentation pour atteindre 70 g/L de biomasse avec le procédé selon l'invention. Cette différence peut s'expliquer par le fait que la souche gère elle-même l'apport en glucose et que son métabolisme n'est par conséquent jamais limité.

De plus, les écarts de concentrations du glucose résiduel sont plus faibles avec le procédé selon l'invention.

### Exemple 2 : Production de Chlorella protothecoides riches en lipides avec ajout de glucose par pulse quand la pO₂ atteint 50 % de la saturation

Le milieu de culture et les conditions opératoires sont identiques à ceux de l'essai 1 de l'exemple 1 :

| | |
|---|---|
| Température | 28 °C |
| pH | 6.8 par NH3 28% w/w puis KOH 5N |
| pO₂ | 30 % (maintenue par agitation) |
| Agitation | 300 RPM mini |
| Débit d'air | 15 L/min |

Comme dans l'exemple 1, les ajouts de glucose sont séquentiels et automatisés grâce à un algorithme contrôlant le fonctionnement de la pompe à partir de la mesure de la teneur en oxygène dissous (pO₂) à l'aide d'une sonde dédiée.

Mais le seuil de pO₂ déclenchant l'ajout de glucose est ici plus élevé : 50 % de la saturation au lieu de 35 % de la saturation.

Soit donc une valeur seuil de déclenchement de 67 % supérieure à la pression en oxygène dissous dans le milieu de fermentation lorsque la concentration en source carbonée dans le milieu de fermentation n'est pas limitante.

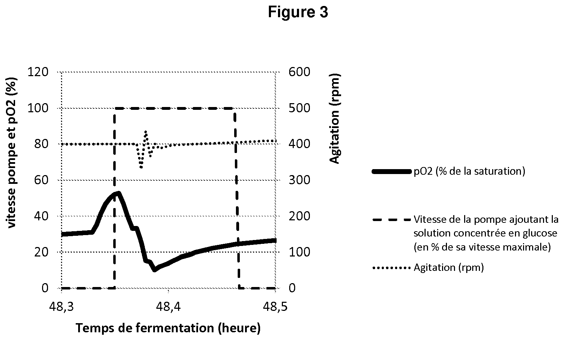

Le graphe présenté dans la Figure 3 donne un exemple de réalisation du pulse. Lorsque la concentration en glucose tombe à une valeur proche de 0 g/L dans le fermenteur, la consommation en oxygène de la souche chute fortement de sorte que la pO₂ remonte rapidement. L'agitation est ici bloquée à 400 rpm.

La détection de la remontée de la pO₂ au-delà de 50 % de la saturation (soit 67% de plus que la valeur avant que le glucose devienne limitant) déclenche la mise en route de la pompe d'alimentation de glucose à sa vitesse maximale.

### Résultats finaux de cet essai :

- Durée : 90 h
- Biomasse : 176 g/L
- Teneur en lipide : 45.1%

Ainsi, malgré l'élévation du seuil de déclenchement de l'ajout (50 % de la saturation par rapport à 35% dans l'exemple 1), les pulses sont effectués très rapidement après l'épuisement du glucose et la durée de culture pour atteindre la concentration en biomasse souhaitée, n'est pas augmentée par rapport à l'exemple 1.

### Description des Figures

- FIGURE 1 :: Alimentation Continu en glucose avec ajustement manuel
- FIGURE 2 :: Alimentation en glucose par ajouts séquentiels et automatiques-valeur seuil de déclenchement : pO2 = 35 % (soit de 16,7 % supérieure à la valeur obtenue lorsque la concentration en source carbonée dans le milieu de fermentation n'est pas limitante).
- FIGURE 3 :: Alimentation en glucose par ajouts séquentiels et automatiques-valeur seuil de déclenchement : pO2 = 50 % (soit de 67 % supérieure à la valeur obtenue lorsque la concentration en source carbonée dans le milieu de fermentation n'est pas limitante).

## Revendications

1. Procédé de production de microalgues du genre *Chlorella* par fermentation en mode discontinu alimenté (dite « fed Batch »), **caractérisé en ce que** l'apport en glucose est réalisé de manière séquentielle et automatique en réponse à une baisse de la consommation en oxygène par la microalgue, dans lequel l'apport en glucose est déclenché lorsque la pression en oxygène dissous dans le milieu de fermentation (pO₂) dépasse une valeur seuil prédéfinie qui est de 10 à 80 % supérieure à la valeur de pO₂ établie lorsque la concentration en glucose dans le milieu de fermentation n'est pas limitante, et dans lequel, lors du déclenchement, le glucose est apporté durant une période de temps de moins de 10 minutes pour atteindre une concentration de 1 à 20 g/L de glucose dans le milieu de fermentation.

2. Procédé selon la revendication 1, dans lequel la valeur de pO₂ établie lorsque la concentration en glucose dans le milieu de fermentation n'est pas limitante, est de 20 à 40%, de préférence d'environ 30%.

3. Procédé selon la revendication 1 ou 2, dans lequel la microalgue du genre *Chlorella* est choisie dans le groupe constitué de *Chlorella protothecoides, Chlorella sorokiniana* et *Chlorella vulgaris.*

4. Procédé selon l'une quelconque des revendications 1 à 3, dans lequel la microalgue est *Chlorella protothecoides.*

5. Procédé selon l'une quelconque des revendications 1 à 4, dans lequel le délai entre le moment où le glucose a été entièrement consommé et un apport en glucose est inférieur à 5 minutes, de préférence inférieur à 1 minute.

6. Procédé selon l'une quelconque des revendications 1 à 5, dans lequel le glucose résiduel est en permanence maintenu à une valeur supérieure à 0 et inférieure à 20 g/L.

7. Procédé selon l'une quelconque des revendications 1 à 6, dans lequel l'apport en glucose est réalisé au moyen d'une pompe dont le débit maximal permet d'ajouter 10 à 20 g/L de glucose au milieu de fermentation en moins de 10 minutes.

## Patentansprüche

1. Verfahren zur Produktion von Mikroalgen der Gattung *Chlorella* mittels diskontinuierlicher Fermentation mit Zufütterung ("Fed-Batch" bezeichnet), **dadurch gekennzeichnet, dass** die Zufuhr von Glucose sequentiell und automatisch als Reaktion auf einen Rückgang des Sauerstoffverbrauchs von der Mikroalge erfolgt, wobei die Zufuhr von Glucose ausgelöst wird, wenn der Druck des im Fermentationsmedium gelösten Sauerstoffs (pO₂) einen zuvor definierten Schwellenwert übersteigt, der 10 bis 80% höher als der ermittelte p02-Wert ist, wenn die Konzentration von Glucose im Fermentationsmedium nicht limitierend ist, und wobei beim Auslösen die Glucose in einem Zeitraum von weniger als 10 Minuten zugeführt wird, um eine Konzentration von 1 bis 20 g/l Glucose im Fermentationsmedium zu erreichen.

2. Verfahren gemäß Anspruch 1, wobei der ermittelte p02-Wert, wenn die Konzentration von Glucose im Fermentationsmedium nicht limitierend ist, 20 bis 40%, vorzugsweise ungefähr 30% beträgt.

3. Verfahren gemäß Anspruch 1 oder 2, wobei die Mikroalge der Gattung *Chlorella* aus der Gruppe ausgewählt ist, bestehend aus *Chlorella protothecoides, Chlorella sorokiniana* und *Chlorella vulgaris.*

4. Verfahren gemäß einem der vorhergehenden Ansprüche 1 bis 3, wobei die Mikroalge *Chlorella protothecoides* ist.

5. Verfahren gemäß einem der Ansprüche 1 bis 4, wobei der Zeitraum zwischen dem Zeitpunkt, wo die Glucose vollständig verbraucht worden ist, und einer Zufuhr von Glucose weniger als 5 Minuten, vorzugsweise weniger als 1 Minute, beträgt.

6. Verfahren gemäß einem der Ansprüche 1 bis 5, wobei die Restglucose ständig auf einem Wert größer 0 und kleiner 20 g/l gehalten wird.

7. Verfahren gemäß einem der Ansprüche 1 bis 6, wobei die Zufuhr von Glucose mithilfe einer Pumpe erfolgt, deren maximale Leistung eine Zufuhr von 10 bis 20 g/l Glucose zum Fermentationsmedium in weniger als 10 Minuten erlaubt.

## Claims

1. A process for the production of microalgae of the *Chlorella* genus by fed-batch fermentation, **characterized in that** the supplying with glucose is carried out sequentially and automatically in response to a fall in the consumption of oxygen by the microalgae, wherein supplying with glucose is triggered when the dissolved oxygen pressure in the fermentation medium (pO₂) exceeds a predefined threshold value which is from 10 to 80% greater than the p02 value established when the concentration of glucose in the fermentation medium is not limiting, and wherein, on triggering, glucose feeding is carried out for a period of time of less than 10 minutes in order to achieve a concentration of glucose in the fermentation medium of 1 to 20 g/L.

2. The process according to claim 1, wherein the pO₂ value established when the concentration of glucose in the fermentation medium is not limiting, is from 20 to 40%, preferably approximately 30%.

3. The process according to claim 1 or 2, wherein the microalga of the *Chlorella* genus is chosen from the group consisting of *Chlorella protothecoides, Chlorella sorokiniana* and *Chlorella vulgaris.*

4. The process according to any one of claims 1 to 3, wherein the microalga is *Chlorella protothecoides.*

5. The process according to any one of claims 1 to 4, wherein the period of time between the moment when the glucose has been completely consumed and supplying with glucose is less than 5 minutes, preferably less than 1 minute.

6. The process according to any one of claims 1 to 5, wherein the residual glucose is permanently maintained at a value of greater than 0 and less than 20 g/L.

7. The process according to any one of claims 1 to 6, wherein supplying with glucose is carried out by means of a pump, the maximum flow rate of which makes it possible to add from 10 to 20 g/L of glucose to the fermentation medium in less than 10 minutes.
